**Europäisches Patentamt**

**European Patent Office**

**Office européen des brevets**

(11) Veröffentlichungsnummer: **0 345 505**
**A2**

## (12) EUROPÄISCHE PATENTANMELDUNG

(21) Anmeldenummer: **89108989.8**

(22) Anmeldetag: **19.05.89**

(51) Int. Cl.4: **B01J 8/04 , C01C 1/04 , C07C 29/15**

(30) Priorität: **08.06.88 DE 3819451**

(43) Veröffentlichungstag der Anmeldung:
**13.12.89 Patentblatt 89/50**

(84) Benannte Vertragsstaaten:
**AT BE CH DE ES FR GB IT LI NL**

(71) Anmelder: **Uhde GmbH**
**Friedrich-Uhde-Strasse 15**
**D-4600 Dortmund 1(DE)**

(72) Erfinder: **Marsch, Hans-Dieter, Dipl.-Ing.**
**Overhoffstrasse 193**
**D-4600 Dortmund 76(DE)**

(54) **Vorrichtung zur Durchführung exothermer, katalytischer Gasreaktionen für die Ammoniak- oder Methanol-Synthese.**

(57) Bei einer Vorrichtung zur Durchführung exothermer, katalytischer Gasreaktionen für die Ammoniak- oder Methanol-Synthese, im wesentlichen bestehend aus einem Hochdruckmantel und einem Einsatz mit oberem Abschlußdeckel, wobei der Einsatz mindestens zwei übereinander angeordnete Katalysatorbehälter (13,16) in ringförmiger zylindrischer Anordnung mit gasdurchlässiger Innen- und Außenwand für radiale Gasströmung von außen nach innen und zwei Gas/Gas-Wärmetauscher (8,12) in Rohrbündelbauart zentral innerhalb des ersten und des zweiten Katalysatorbehälters enthält, soll eine Lösung geschaffen werden, wodurch auf ein zentrales Leitrohr verzichtet werden kann.

Dies wird dadurch erreicht, daß beide Gas/Gas-Wärmetauscher rohrseitig als zweiflutig ausgebildet sind, derart daß der erste Wärmetauscher (8) an das zentrale Frischgaszuführrohr (6) angeschlossen ist, die zentralen Wärmetauscherrohre mittels der Zwischenkammer (10) mit den zentralen Wärmetauscherrohren des zweiten Wärmetauschers (12) verbunden sind, daß die peripheren Wärmetauscherrohre des zweiten Wärmetauschers (12) über die Zwischenkammer (14) mit den peripheren Wärmetauscherrohren des ersten Wärmetauschers verbunden sind und die peripheren Wärmetauscherrohre im Freiraum oberhalb des Wärmetauschers münden.

Fig. 1

## Vorrichtung zur Durchführung exothermer, katalytischer Gasreaktionen für die Ammoniak- oder Methanol-Synthese

Die Erfindung betrifft eine Vorrichtung zur Durchführung exothermer, katalytischer Gasreaktionen für die Ammoniak- oder Methanol-Synthese, im wesentlichen bestehend aus einem Hochdruckmantel und einem Einsatz mit oberem Abschlußdeckel, wobei der Einsatzmantel mindestens zwei übereinander angeordnete Katalysatorbehälter in ringförmiger zylindrischer Anordnung mit gasdurchlässiger Innen- und Außenwand für radiale Gasströmung von außen nach innen und zwei Gas/Gas-Wärmetauscher in Rohrbündelbauart zentral innerhalb des ersten und des zweiten Katalysatorbehälters enthält und wobei beide Gas/Gas-Wärmetauscher rohrseitig vom Frischgas und mantelseitig vom Reaktionsgas durchströmt werden.

Die katalytische Hochdrucksynthese zur Erzeugung von Ammoniak und Methanol ist ein exothermer Prozeß. Man ist seit jeher bestrebt, diesen Prozeß bei solchen Temperaturen ablaufen zu lassen, daß die thermischen Verhältnisse zu optimaler Reaktion, d.h. zu optimalem Umsatz, führen. Die Einhaltung der günstigsten Reaktionstemperaturen geschieht durch Abkühlung der bei der Reaktion sich stark erwärmenden Reaktionsgase. Da das Frischgas mit einer Temperatur von über 300 °C auf die 1. Katalysatorschicht gegeben werden muß, wird angestrebt, das Frischgas mit dem heißen Reaktionsgas in indirektem Wärmetausch aufzuheizen.

Die Abkühlung des Reaktionsgases kann nun entweder so erfolgen, daß die Reaktion fast isothermisch verläuft, wie es bei Katalysatorröhrenöfen oder Vollraumöfen mit Kühlschlangen in der Katalysatormasse der Fall ist, oder so, daß die Abkühlung stufenweise nach einzelnen Katalysatorpackungen erfolgt, in die die gesamte Katalysatormasse aufgeteilt ist.

Es sind zahlreiche Vorrichtungen bzw. Ofentypen bekannt zur Lösung der o.g. Forderungen. Es ist beispielsweise bekannt, die Katalysatormasse in mehreren Lagen untereinander anzuordnen und in den Zwischenräumen Röhren-Wärmetauscher für aufzuheizendes Frischgas einzubauen, um die Reaktionstemperaturen zu senken.

Nach DE-OS 33 43 114 ist ein Radial-Reaktor mit zentral angeordneten Zwischenwärmetauschern bekannt. Die beiden Zwischenwärmetauscher weisen in etwa gleiche Außendurchmesser auf und ihre, mit geringem Abstand zueinander liegenden beiden inneren Rohrböden, sind mittels eines Mantelbleches gasdicht verbunden. Über ein zentrales Leitrohr besteht eine durchgängige Verbindung vom Freiraum zwischen Hochdruckmantel und Einsatz zum unteren Rohrboden des unteren Wärmetauschers. Dieses zentrale Leitrohr benötigt Hochdruckraum.

Der Erfindung liegt die Aufgabe zugrunde, auf das zentrale Leitrohr zu verzichten.

Die Aufgabe wird erfindungsgemäß gelöst durch eine Vorrichtung zur Durchführung exothermer, katalytischer Gasreaktionen für die Ammoniak- oder Methanol-Synthese mit den Merkmalen entsprechend den konstruktiven Ausgestaltungen gemäß den Kennzeichen des Hauptanspruches.

Nach einer Ausgestaltung der Erfindung kann der zweite Wärmetauscher auch eine Zufuhrleitung für Zumischgas zum aufzuheizenden Frischgas enthalten.

Nach weiteren Ausgestaltungen sind die Rohre des zweiten Wärmetauschers Haarnadelrohre bzw. münden sie einseitig in einer Rohrbodenkammer.

Die nachfolgend anhand der Abbildungen ausführlich beschriebene erfindungsgemäße Vorrichtung erlaubt einen optimalen Ausnutzungsgrad des Hochdruckraumes mittels Wärmetauscherfläche.

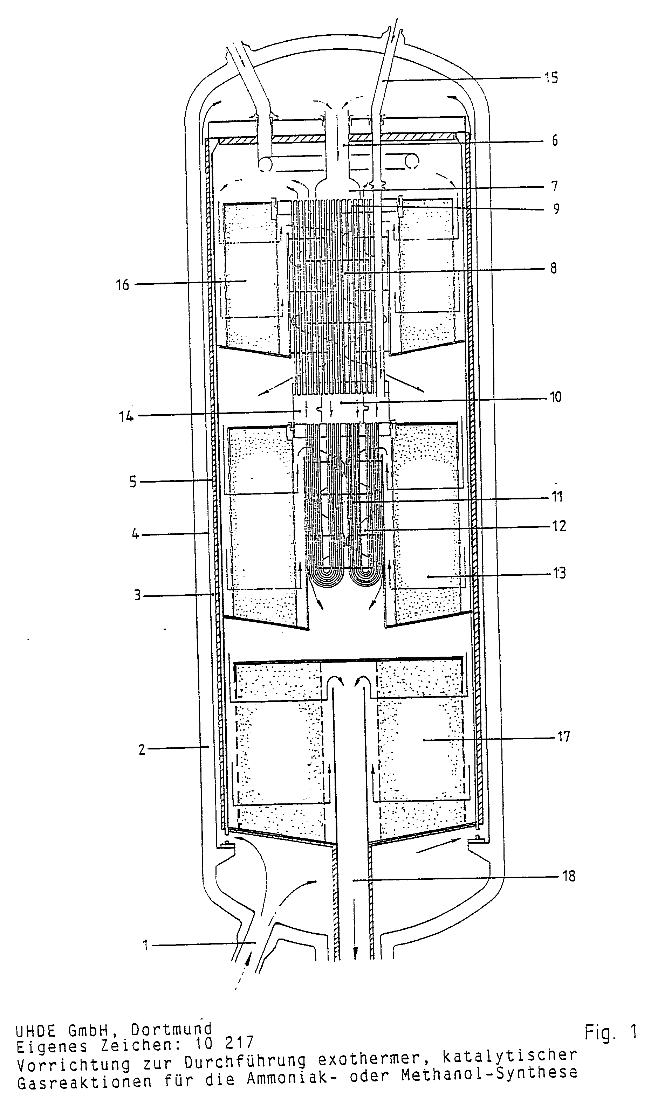

Ein Ausführungsbeispiel der Erfindung ist in der Fig. 1 dargestellt und wird im folgenden näher beschrieben.

Das Synthesegas tritt über den Gaseinlaß 1 in den Konverter 2 ein und strömt in dem Ringraum 3 zwischen der Konverterwand 4 und der Wand des Konverteinsatzes 5 aufwärts, um über das Frischgaszuführrohr 6 in die Eintrittskammer 7 des ersten Wärmetauschers 8 zu gelangen. Über die Eintrittskammer wird nur ein Teil der oberen Rohrbodenplatte 9, und zwar der Zentrale, beaufschlagt. Das aufzuheizende Synthesegas durchströmt nun die inneren Rohre des ersten Wärmetauschers 8 und wird dabei von 240 auf 311 °C aufgeheizt, sammelt sich in der Zwischenkammer 10 und durchströmt nun die Innenschenkel der im Beispiel gewählten Haarnadel-Rohre 11 des zweiten Wärmetauschers 12. Am Ende der Innenschenkel wird eine Temperatur von etwa 340 °C erreicht. Die Gleichstromanteile der beiden Wärmetauscher 8 und 12 bringen somit eine Temperaturerhöhung von 100 °C. Danach strömt das Gas in den Außenschenkel des zweiten Wärmetauschers 12 im Gegenstrom zum heißeren Gas aus dem zweiten Katalysatorbett 13 zurück und gelangt in die äußere Zwischenkammer 14 mit einer Temperatur von 375 °C. Gleichzeitig mündet in diese Kammer ein Bypassrohr 15, womit die vorerwähnte Temperatur veränderbar ist. Von dieser Zwischenkammer 14 strömt das Gas in die Peripherierohre des ersten Wärmetauschers im Gegenstrom zu dem heißeren Gas aus dem ersten Katalysatorbett 16. Am Austritt aus dem ersten Wärmetauscher hat das Synthesegas eine Temperatur von 396 °C, mit der es dem ersten Katalysatorbett 16 zugeführt wird, das es radial von außen nach innen durchströmt. Durch die exotherme Reaktion der Ammoniakbildung erhöht sich dabei die Temperatur. Die entstehende Wärme wird über den ersten Wärmetauscher 8 abgeführt. Dabei strömt das zu kühlende Synthesegas mantelseitig. Den Pfeilen folgend wiederholt sich der Vorgang über das zweite Katalysatorbett und den zweiten Wärmetauscher, wodurch die NH₃-Produktion wieder steigt. Nach mantelseitiger Durchströmung des zweiten Wärmetauschers wird das Synthesegas einem dritten Katalysatorbett 17 zugeführt, um einen maximalen $NH_3$-Gehalt zu erzielen. Die erneute Temperatursteigerung wird jetzt aber nicht mehr durch einen Gas/Gas-Wärmetauscher abgebaut wie nach den beiden ersten Betten, sondern das Synthesegas mit maximalem $NH_3$-Gehalt wird über das Auslaßrohr 18 aus dem Konverter abgezogen und sein Wärmeinhalt wird in einem nachgeschalteten Apparat, beispielsweise zur Dampferzeugung, ausgenutzt.

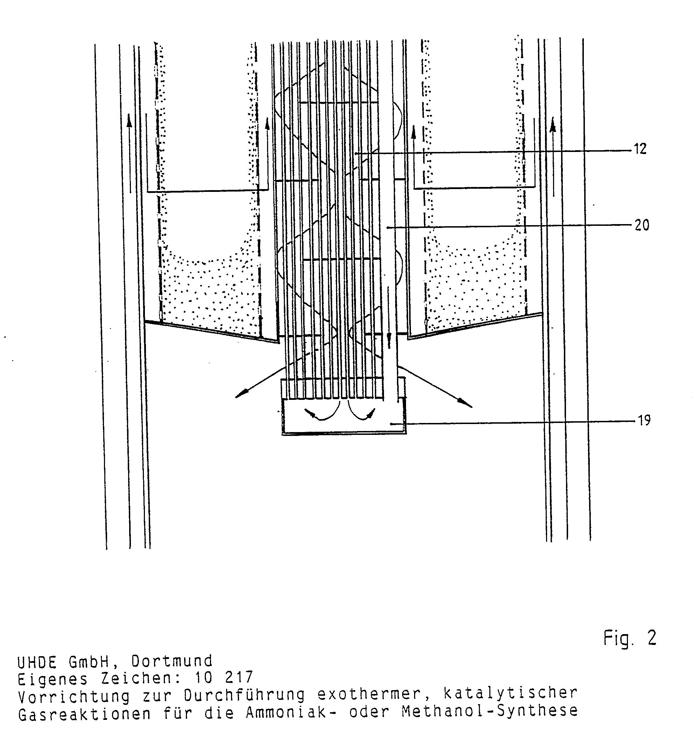

In Fig. 2 werden im zweiten Wärmetauscher 12 anstelle von Haarnadelrohren normale Einzelrohre verwendet. Die Umleitung des in den zentralen Rohren abströmenden, aufzuheizenden Frischgases in die peripheren Rohre erfolgt mittels der Rohrbodenkammer 19 im unteren Teil des Wärmetauschers, in die ebenfalls, wie beispielhaft dargestellt, ein Bypassrohr II (20) zur Regelung der Temperatur münden kann.

Die Vorteile der erfindungsgemäßen Strömungsführung liegen in der Vermeidung eines zentralen Gasleitrohres und damit in der Erzielung einer optimalen Ausnutzung des Hochdruckraumes.

## Ansprüche

1. Vorrichtung zur Durchführung exothermer, katalytischer Gasreaktionen für die Ammoniak- oder Methanol-Synthese, im wesentlichen bestehend aus einem Hochdruckmantel und einem Einsatz mit oberem Abschlußdeckel, wobei der Einsatz mindestens zwei übereinander angeordnete Katalysatorbehälter (13,16) in ringförmiger zylindrischer Anordnung mit gasdurchlässiger Innen- und Außenwand für radiale Gasströmung von außen nach innen und zwei Gas/Gas-Wärmetauscher (8,12) in Rohrbündelbauart zentral innerhalb des ersten und des zweiten Katalysatorbehälters enthält, wobei beide Gas/Gas-Wärmetauscher rohrseitig vom Frischgas und mantelseitig vom Reaktionsgas durchströmt werden,
**dadurch gekennzeichnet,**
daß beide Gas/Gas-Wärmetauscher rohrseitig als zweiflutig ausgebildet sind, derart daß der erste Wärmetauscher (8) an das zentrale Frischgaszuführrohr (6) angeschlossen ist, die zentralen Wärmetauscherrohre mittels der Zwischenkammer (10) mit den zentralen Wärmetauscherrohren des zweiten Wärmetauschers (12) verbunden sind, daß die peripheren Wärmetauscherrohre des zweiten Wärmetauschers (12) über die Zwischenkammer (14) mit den peripheren Wärmetauscherrohren des ersten Wärmetauschers verbunden sind und die peripheren Wärmetauscherrohre im Freiraum oberhalb des Wärmetauschers münden.

2. Vorrichtung nach Anspruch 1,
**gekennzeichnet durch**
ein Bypassrohr (15) für Zumischgas, die durch den ersten Gas/Gas-Wärmetauscher bis in die Zwischenkammer (14) führt.

3. Vorrichtung nach Anspruch 1,
**dadurch gekennzeichnet,** daß die zentralen und peripheren Wärmetauscherrohre des 2. Wärmetauschers (12) als Haarnadelrohre ausgebildet sind.

4. Vorrichtung nach Anspruch 1 und 3,
**dadurch gekennzeichnet,** daß die zentralen und peripheren Wärmetauscherrohre des 2. Wärmetauschers (12) in der Rohrbodenkammer (19) münden.

5. Vorrichtung nach Anspruch 1, 3 und 4,
**dadurch gekennzeichnet,** daß die zentralen und die peripheren Wärmetauscherrohre unterschiedliche Innendurchmesser aufweisen.

UHDE GmbH, Dortmund
Eigenes Zeichen: 10 217
Vorrichtung zur Durchführung exothermer, katalytischer
Gasreaktionen für die Ammoniak- oder Methanol-Synthese

Fig. 1

Fig. 2

UHDE GmbH, Dortmund
Eigenes Zeichen: 10 217
Vorrichtung zur Durchführung exothermer, katalytischer
Gasreaktionen für die Ammoniak- oder Methanol-Synthese